(19) Europäisches Patentamt

European Patent Office

Office européen des brevets

(11) EP 0 708 070 B1

(12) EUROPEAN PATENT SPECIFICATION

(45) Date of publication and mention
of the grant of the patent:
**06.05.1999 Bulletin 1999/18**

(51) Int. Cl.$^6$: **C07C 7/13**

(21) Application number: **95114721.4**

(22) Date of filing: **19.09.1995**

(54) **Process for the separation of paraffins mixed with olefins**

Verfahren zur Trennung von mit Olefinen gemischten Paraffinen

Procédé pour la séparation de paraffines mélangées à des oléfines

(84) Designated Contracting States:
**BE DE ES FR GB NL**

(30) Priority: **19.10.1994 IT MI942134**

(43) Date of publication of application:
**24.04.1996 Bulletin 1996/17**

(73) Proprietor: **ENICHEM S.p.A.**
**20124 Milano (IT)**

(72) Inventors:
 • **Paludetto, Renato**
  **Pioltello (MI) (IT)**
 • **Donati, Gianni**
  **RHO (MI) (IT)**
 • **Orsi, Alfredo**
  **Corsico (MI) (IT)**
 • **Pandolfi, Gianni**
  **Novara (IT)**

(74) Representative:
 **Gennari, Marco, Dr.**
 **EniTecnologie S.p.A.**
 **Patent and Licensing Dept.**
 **Via F. Maritano, 26**
 **20097 San Donato Milanese (MI) (IT)**

(56) References cited:
 **EP-A- 0 572 239**       **DD-A- 150 888**

 • **CHEMICAL ABSTRACTS, vol. 97, no. 19, 8**
  **November 1982 Columbus, Ohio, US; abstract**
  **no. 162327s, page 657; & SU-A-943 218**
  **(MAMEDALIEV ET AL)**

## Description

The present invention relates to a process for the separation of paraffins mixed with olefins.

More specifically, the present invention relates to a process for the separation of paraffins mixed with olefins having the same number of carbon atoms, carried out in a vapour phase and in the presence of molecular sieves.

Still more specifically, the present invention relates to a process for the separation of butanes or pentanes mixed with butenes and pentenes respectively.

It is known that the separation of a paraffin contained in a mixture with the corresponding olefin, or having the same number of carbon atoms, is a not an easy operation to carry out. A consolidated method is by distillation, for example fractional distillation with a plate tower. This technique however, as the products to be separated have boiling points which are very close to each other, requires the use of distillation columns with a large number of fractionation plates and this leads not only to high investment costs but also to substantial operating costs. Moreover, in the case of the separation of n-pentane from n-pentenes, fractional distillation is not possible as n-pentane and trans-pentene-2 basically have the same boiling point.

An alternative method for separating a paraffin contained in a mixture with the corresponding olefin is by adsorption/desorption on molecular sieves carried out in a liquid phase. This technique, described for example in the text "Preparative and Production Scale Chromatography" by G. Ganetsos and P.E. Baker, page 263 onwards, exploits the capacity of selective adsorption on the part of solid adsorbing materials with respect to substances to be separated and allows the two components of the mixture to be separated with a high degree of purity but requires the use of sophisticated equipment with consequent high investment and operating costs.

As already mentioned, the known methods for separating a paraffin mixed with the corresponding olefin allow two very distinct fractions to be obtained with a high degree of purity, one basically consisting of the paraffin and the other of the corresponding olefin. These fractions are thus available for possible, subsequent transformations.

There are organic synthesis processes however which involve hydrocarbon fractions like those mentioned above for which such a sophisticated separation is not indispensable but can be limited to only one of the two fractions.

For example, processes are known in the art for the production of alkyl ter-butylic ethers which consist in reacting the isobutene, contained in $C_4$ hydrocarbon streams of a different origin, in particular in streams coming from steam cracking or catalytic cracking plants, or plants for the dehydrogenation of isobutane, with an alcohol, preferably selected from methanol and ethanol. U.S. patents 3.979.461, 4.039.590 and 4.071.567, for example, describe several processes for the preparation of methyl ter-butylic ether (MTBE) in which the isobutene, contained in $C_4$ streams possibly also comprising butadiene, is reacted with methanol in the presence of an acid ion exchange resin. Other processes for the synthesis of MTBE are described in U.S. patent 4.475.005 or in the published European patent application 470.655.

According to these processes it is possible to obtain an almost total transformation of the isobutene whereas the other olefinic components remain practically unchanged and must be recovered for their evaluation.

English patent 2.121.407 and U.S. patent 4.513.153, for example, describe a process for the production of alkyl ter-butylic ethers which comprises a cycle for the evaluation of the residual components still present in the $C_4$ stream leaving the etherification reactor. According to these patents, the $C_4$ stream, after the synthesis and separation from the ter-butylic ether, is sent to a sketeton isomerization unit, for the conversion of the butene-1 and butenes-2, cis and trans, to isobutene, and is recycled to the synthesis section of alkyl ter-butylic ether. As there is, in such an integrated system, an undesired accumulation of inert products, basically consisting of saturated hydrocarbons such as n-butane and isobutane, a fractionation or extractive distillation section of the $C_4$ hydrocarbon stream has been inserted between the etherification and isomerization sections, to separate the saturated hydrocarbons from the unsaturated ones.

The integrated process for the production of alkyl ter-butylic ethers of the known art has the disadvantage, however, of having to run the fractionation or extractive distillation section which, inevitably, significantly influences and raises the investment and production costs.

The alternative to the present process is to carry out a simple blowdown. Also in this case, however, the solution has its disadvantages as, together with the inert products, unsaturated hydrocarbons are purged, which are basically butenes 1 and 2, which, for the integrated cycle, constitute valuable material to be evaluated.

U.S. patent 4.718.986, on the other hand, describes a process for the separation of butene-1, contained in a $C_4$ stream, by means of a distillation unit. According to this patent, in a first column, the separation is carried out at the head of the isobutane which would otherwise pollute the end-product butene-1 recovered with a high purity (>99%) as product at the head of a second distillation column. The quantity of isobutane present in the stream influences not only investments and consumption relating to the first column, but also the total yield of the butenes as it withholds substantial quantities of butene-1 (on an average in the ratio isobutane/butene-1 of 1/15) in the blowdown at the head of the first column.

Also in this case, the separation of the butene-1 occurs with significant losses of valuable material in the blowdown at the head of the first distillation column.

In the above processes, and in others such as, for example, the integrated process for the synthesis of alkyl ter-amylic ethers (TAME) from $C_5$ streams, the recovery of the valuable olefin fraction from the blowdowns does not require high purity which, on the other hand, is necessary for the paraffin fraction, generally destined to the flare or co-cracking (for the $C_4$ products) and gasoline pool (for the $C_5$ products).

EP 572 239 discloses a process for the separation of hydrocarbon mixtures by using the pressure swing adsorption technology.

The Applicant has now found a new process for the separation of paraffins mixed with olefins having the same number of carbon atoms, which allows only the paraffin fraction to be obtained with a high purity and uses equipment with low investment and operating costs. The olefin fraction, on the other hand, although still containing considerable traces of paraffin can be used for the organic syntheses which do not require particular conditions of purity. This result can be obtained if the separation is carried out on molecular sieves feeding the mixture to be treated in a vapour phase, as operating in a liquid phase, as subsequently shown, gives unsatisfactory results.

The present invention therefore relates to a process for the separation of paraffins mixed with olefins having the same number of carbon atoms which comprises:

a) feeding the mixture in a vapour phase to an adsorption unit operating with molecular sieves capable of having selectivity with respect to the double bond;
b) discharging the paraffin basically without olefin;
c) desorbing the olefin from the molecular sieves by elution with a desorbing agent in a vapour phase;
d) recovering the olefin, mixed with the desorbing agent, by distillation.

According to the present separation process, the recovered olefins still contain significant quantities of paraffins as during step (a) portions of paraffins remain inside the adsorption unit partly occupying the interstitial volume of the molecular sieves and partly also being adsorbed in the sieves. In the desorption phase, these portions of paraffin are drawn by the desorbing agent together with the olefins and remain mixed with these.

Analogously, at the end of the desorption, quantities of desorbing agent remain in the interstices of molecular sieves and are drawn by the paraffins during the adsorption step (a). If these quantities of desorbing agent are to be recovered, it is possible to carry out a simple distillation operation.

When the vapour phase is used, the paraffinic residue present in the adsorption unit and occupying the interstitial volume, is limited and much less compared to the operation in a liquid phase. In addition, the operation in a liquid phase requires a much higher number of theoretical steps, with respect to the process in a vapour phase, owing to the fact that the diffusion in a vapour phase are much higher than in a liquid phase. On the contrary, the quantities of product adsorbed by the sieves and consequently the adsorption capacity per volume unit is the same operating both the in the liquid and vapour phase.

This fact makes the process in a vapour phase much simpler and more economical and, when high purities in the olefin stream are not required, as in the above applications (MTBE and TAME), results in extreme simplification of the plant and low incidence on the investment and operating costs of the integrated process.

Examples of paraffin-olefin mixtures which can be used in the process of the present invention are mixtures basically consisting of ethane-ethylene, propane-propylene, butanes-butenes, pentanes-pentenes, etc.

Any molecular sieve of the zeolite type capable of having selectivity with respect to the olefinic double bond can be used in the process of the present invention even if X and Y type zeolites are preferred with a particle size of between 0.1 and 3 mm. These zeolites allow selectivity ratios olefins/paraffins of between 3 and 12 to be obtained, the selectivity being defined as:

$$S = \frac{\Gamma_o/P_o}{\Gamma_p/P_p}$$

wherein $\Gamma_o$ and $\Gamma_p$ are the adsorbed molar quantities of olefins (o) and paraffins (p) in equilibrium with the respective partial pressures $P_o$ and $P_p$ in the vapour.

The separation of the paraffins according to the present process is carried out in a vapour phase at a temperature of between -10 and 180°C, preferably between 20 and 140°C, and a pressure of between 1 and 10 absolute bars, preferably between 1 and 5. To guarantee continuity to the process it is possible to use a system with at least two adsorption units situated parallel to each other so that when one section is in the adsorption phase the other is in the desorption phase.

The desorption is carried out by elution of the olefins adsorbed on the molecular sieves with a desorbing agent. Preferred desorbing agents are those with a boiling point which is significantly different from that of the components of the mixture to be treated. For example the desorbing agents can be selected from aliphatic hydrocarbons such as pentane, hexane, heptane, octane, etc.

The process of the present invention allows a paraffin stream to be obtained, practically without olefins, which is not very valuable, and an olefin stream which is more valuable and which, with the desired purities and possibly still containing quantities of paraffins, can be used in those organic synthesis processes where the use of a high purity olefin fraction is not indispensable.

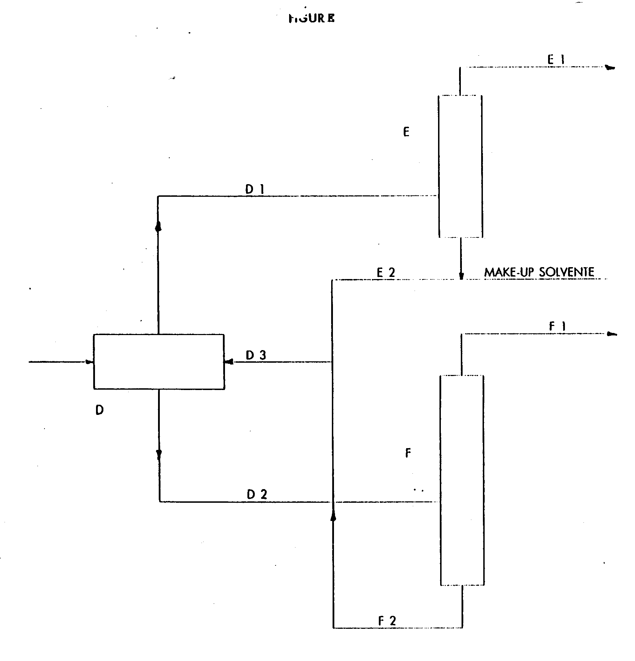

The process for the separation of paraffins mixed with olefins having the same number of carbon atoms of the present invention can be better illustrated with reference to the block sketch of the enclosed figure which represents an illustrative form of embodiment of the invention.

With reference to the figure, the apparatus with which the separation is carried out comprises an adsorption/desorption unit D and two distillation columns E and F.

To operate in continuous it is also possible to have two units D operating alternatively one in the adsorption phase and the other in the desorption phase.

Two streams D1 and D2 are recovered from unit D.

In the adsorption phase stream D1 is recovered, practically without olefins, and is sent to the distillation column E for the recovery of the desorbing agent E2, which is recycled, from the blowdown paraffin fraction E1.

In the desorption phase stream D2 is recovered, basically consisting of the adsorbed olefins and paraffins remaining inside unit D. This stream is sent to the distillation column F for the recovery of the olefin fraction F1, also containing the residual paraffins, from the desorbing agent F2 which is recycled.

Some illustrative examples are given below to provide a better understanding and for the embodiment of the present invention.

EXAMPLE 1

Samples of type X zeolite in the form of 1/16" pellets are heated in muffle at 400°C for 5 hours in a nitrogen stream.

4 g of zeolite thus treated are charged into an AISI 316 steel column about 25 cm long which is placed in an oven and brought to a temperature of 90°C.

Once this temperature value has been reached, a stream of n-hexane vapours is passed inside the tube for about 1000 seconds to saturate the active sites of the zeolite. A $C_4$ stream, in a vapour phase, is then passed with a flow rate of 7.6 l/h.

The composition of the $C_4$ stream is the following:

|  | weight % |
|---|---|
| - i-butane | = 3.8 |
| - i-butene | = 4.4 |
| - n-butane | = 11.2 |
| - n-butenes | = 80.0 |
| - 1,3-butadiene | = 0.6 |

The $C_4$ stream is passed inside the column for a period of about 1100 seconds. The flow is then stopped and a stream of n-hexane in a vapour phase is then fed with a flow rate of 0.6 cc/min for about 1800 seconds.

The effluent leaving the system is condensed in a glass heat exchanger at -15°C.

The recovered mixture consists of the sum of the adsorbed quantities of the $C_4$ load and the quantities contained in the volume of the system not occupied by the zeolite.

The composition of the $C_4$ mixture recovered at the outlet of the column is the following:

|  | weight % |
|---|---|
| - i-butane | = 1.15 |
| - i-butene | = 4.78 |
| - n-butane | = 3.46 |
| - n-butenes | = 88.77 |
| - 1,3-butadiene | = 1.84 |

The experimental data and gaschromatographic analysis of the condensed liquid show the following selectivities of the system:

| i-butene/n-butane | = 3.60 |
|---|---|
| butene-1/n-butane | = 3.70 |
| t-butene-2/n-butane | = 2.76 |
| c-butene-2/n-butane | = 4.13 |

As the selectivity of the system, $S_i$, the following ratio is intended:

$$S_i = \frac{A_o/A_i}{R_o/R_i}$$

wherein:

- A and R are, respectively, the molar fractions of the vapour fed (or liquid for the following comparative example) and those of that recovered;
- i and o are, respectively, the general component and reference component (n-butane).

EXAMPLE 2

The same procedure is carried out as for example 1, except that a zeolite Y is used instead of the zeolite X. The flow rate of the $C_4$ stream is equal to 0.5 cc/min.

The composition of the $C_4$ mixture recovered at the outlet of the column is the following:

|  | weight % |
|---|---|
| - i-butane | = 1.00 |
| - i-butene | = 6.44 |
| - n-butane | = 3.87 |
| - n-butenes | = 87.38 |
| - 1,3-butadiene | = 1.31 |

The experimental data and gaschromatographic analysis of the condensed liquid show the following selectivities of the system:

| i-butene/n-butane | = 4.33 |
|---|---|
| butene-1/n-butane | = 3.00 |
| t-butene-2/n-butane | = 3.22 |
| c-butene-2/n-butane | = 5.30 |

COMPARATIVE EXAMPLE

The same procedure is carried out as for example 2 except that the $C_4$ mixture and hexane in a liquid phase are used. To obtain this result the operating pressure inside the system is 14 bar.

The composition of the $C_4$ mixture recovered at the outlet of the column is the following:

|  | weight % |
|---|---|
| - i-butane | = 3.63 |
| - i-butene | = 4.45 |
| - n-butane | = 10.66 |
| - n-butenes | = 80.62 |
| - 1,3-butadiene | = 0.64 |

The experimental data and gaschromatographic analysis of the condensed liquid show the following selectivities of the system:

| i-butene/n-butane | = 1.08 |
|---|---|
| butene-1/n-butane | = 1.05 |
| t-butene-2/n-butane | = 1.06 |
| c-butene-2/n-butane | = 1.11 |

It can therefore be observed that the selectivity of the system is considerably lower than that of the system in a vapour phase making this process configuration in a liquid phase almost impossible.

**Claims**

1. Process for the separation of paraffins mixed with olefins having the same number of carbon atoms which comprises:

   a) feeding the mixture in a vapour phase to an adsorption unit operating with molecular sieves capable of having selectivity with respect to the double bond;
   b) discharging the paraffin basically without olefin;
   c) desorbing the olefin from the molecular sieves by elution with a desorbing agent in a vapour phase;
   d) recovering the olefin, mixed with the desorbing agent, by distillation.

2. Process according to claim 1, wherein the paraffin-olefin mixtures are selected from those basically consisting of ethane-ethylene, propane-propylene, butanes-butenes, pentanes-pentenes.

3. Process according to claim 1 or 2, wherein the molecular sieves are selected from X and Y type zeolites with a particle size of between 0.1 and 3 mm.

4. Process according to any of the previous claims, wherein the separation of the paraffins takes place in a vapour phase at a temperature of between -10 and 180°C, and at a pressure of between 1 and 10 absolute bars.

5. Process according to any of the previous claims, wherein the desorbing agents are those with a boiling point which is different from that of the components of the mixture to be treated.

**Patentansprüche**

1. Verfahren zur Abtrennung von Paraffinen, die mit Olefinen mit der gleichen Anzahl an Kohlenstoffatomen vermischt sind, welches umfaßt:

    a) Zufuhr der Mischung in einer Dampfphase zu einer Adsorptionseinheit, welche mit Molekularsieben arbeitet, die in der Lage sind, Selektivität gegenüber der Doppelbindung zu zeigen;
    b) Austrag des Paraffins im wesentlichen ohne Olefin;
    c) Desorption des Olefins aus den Molekularsieben durch Eluieren mit einem Desorptionsmittel in einer Dampfphase;
    d) Gewinnung des mit dem Desorptionsmittel gemischten Olefins durch Destillation.

2. Verfahren nach Anspruch 1, worin die Paraffin-Olefin-Mischungen aus jenen ausgewählt sind, die im wesentlichen aus Ethan-Ethylen, Propan-Propylen, Butanen-Butenen, Pentanen-Pentenen bestehen.

3. Verfahren nach Anspruch 1 oder 2, worin die Molekularsiebe ausgewählt sind aus Zeolithen vom Typ X und Y mit einer Teilchengröße zwischen 0,1 und 3 mm.

4. Verfahren nach irgendeinem der vorhergehenden Ansprüche, worin die Abtrennung der Paraffine in einer Dampfphase bei einer Temperatur zwischen -10°C und 180°C und einem absoluten Druck zwischen 1 und 10 bar stattfindet.

5. Verfahren nach irgendeinem der vorhergehenden Ansprüche, worin die Desorptionsmittel jene sind, deren Siedepunkt sich von dem der Komponenten der zu behandelnden Mischung unterscheidet.

**Revendications**

1. Procédé de séparation de paraffines mélangées à des oléfines ayant un nombre identique d'atome de carbone, lequel procédé comprend les étapes consistant :

    a) à alimenter une unité d'adsorption fonctionnant avec des tamis moléculaires présentant une sélectivité vis-à-vis de la double liaison, avec le mélange en phase vapeur ;
    b) à évacuer la paraffine essentiellement exempte d'oléfine ;
    c) à désorber l'oléfine du tamis moléculaire par élution avec un agent désorbant en phase vapeur ;
    d) à récupérer l'oléfine, mélangée à l'agent désorbant, par distillation.

2. Procédé selon la revendication 1, dans lequel les mélanges paraffine-oléfine sont choisis parmi ceux comprenant essentiellement de l'éthane et de l'éthylène, du propane et du propylène, des butanes et des butènes, des pentanes et des pentènes.

3. Procédé selon la revendication 1 ou 2, dans lequel le tamis moléculaire est choisi parmi les zéolithes de type X et Y constituées de particules ayant une taille comprise entre 0,1 et 3 mm.

4. Procédé selon l'une quelconque des revendications précédentes dans lequel la séparation des paraffines a lieu en phase vapeur à une température comprise entre -10 et 180 °C et à une pression absolue comprise entre 1 et 10 bars.

5. Procédé selon l'une quelconque des revendications précédentes dans lequel les agents désorbants sont ceux ayant un point d'ébullition différent de celui des composants du mélange à traiter.

E 1

E

D 1

E 2          MAKE-UP SOLVENTE

F 1

D 3

D

F

D 2

F 2

EP 0 708 070 B1